# EUROPÄISCHE PATENTSCHRIFT

(11) **EP 0 389 778 B1**
(45) Veröffentlichungstag und Bekanntmachung des Hinweises auf die Patenterteilung: **04.05.1994**
(21) Anmeldenummer: 90102973.6
(22) Anmeldetag: 15.02.1990
(51) Int. Cl.: A61K 7/043

(54) **Nagellack**
Nail Varnish
Vernis à ongles

(30) Priorität: 24.02.1989 CH 690/89; 09.11.1989 CH 4045/89
(43) Veröffentlichungstag der Anmeldung: 03.10.1990
(73) Patentinhaber: F. HOFFMANN-LA ROCHE AG, 4002 Basel (CH)
(72) Erfinder: Ferro, Alberto, Dr., CH-4125 Riehen (CH); Gerhards, Jürgen, Dr., CH-4144 Arlesheim (CH); Werner, Roland, Dr., CH-4045 Basel (CH)
(74) Vertreter: Lederer, Franz, Dr.

(56) Entgegenhaltungen:
- GB-A- 2 107 186
- GB-A- 2 188 844
- GB-A- 2 202 743
- STN INTERNATIONAL DATENBANK ANBIETER, Karlsruhe BRD, DATENBANK CHEMICAL ABSTRACTS & CHEMICAL ABSTRACTS, Band 108, Nr. 10, Zusammenfassung Nr. 82099z, Columbus, Ohio, US; & JP-A-62 123 112 (SHOWA) 04-06-1987
- STN INTERNATIONAL, DATENBANK ANBIETER, Karlsruhe, BRD, DATENBANK CHEMICAL ABSTRACTS & CHEMICAL ABSTRACTS, Band 110, Nr. 14, Zusammenfassung Nr. 121426s, Columbus, Ohio, US; & JP-A-63 130 541 (SHOWA) 02-06-1988 (Kat. A)

## Beschreibung

Die vorliegende Erfindung betrifft ein antimykotisch wirksames Präparat in Form eines Nagellacks, welches mindestens eine antimykotisch wirksame Substanz und mindestens einen wasserunlöslichen Filmbildner enthält. Die Erfindung betrifft ferner ein Verfahren zur Herstellung eines derartigen Präparates.

Aus der UK-Patentpublikation 2 202 743 A sind wässrige antimykotische Präparate zur Behandlung von Pilzinfektionen von Nägeln und/oder der umgebenden Haut bekannt, z.B. zur Behandlung der die Nägel befallenden Pilzinfektion Onychomycose. Diese Präparate enthalten als Wirkstoff Miconazol- oder Econazolnitrat und u.a. ein wasserlösliches Solubilisierungshilfsmittel, z.B. Aethanol, und können u.a. in Form eines Nagellacks vorliegen. Im Falle des Nagellacks soll das Solubilisierungshilfsmittel einen ebenfalls vorhandenen Filmbildner derart solubilisieren, dass nach Verdunsten oder Penetration ein Filmüberzug auf dem Nagel gebildet wird. Unter den gemäss dieser Patentpublikation anwendbaren Filmbildnern (Harzen) wird Eudragit® E 100 erwähnt, das ein Copolymerisat aus Methacrylsäuredimethylaminoäthylester und Methacrylsäure-niederalkylester(n) ist. Es sind in derartigen Copolymerisaten keine quartären Ammoniumgruppen vorhanden.

Ziel der Erfindung ist eine Verbesserung der Qualität von Präparaten der oben genannten Art, und zwar insbesondere hinsichtlich der folgenden Eigenschaften: Gute Penetrationsrate, gute Hautverträglichkeit, gutes Fliessverhalten, gute Verstreichbarkeit, kurze Eintrocknungszeit, nur mässig hoher Glanz, genügend grosse Härte und lange Verweildauer (etwa 3-4 Tage).

Dieses Ziel wird erfindungsgemäss dadurch erreicht, dass man als antimykotisch wirksame Substanz ein Salz des 4-[3-[p-(α,α-Dimethyl-benzyl)-phenyl]-2-methyl-propyl]-2,6-dimethyl-morpholins oder des 4-[3-[p-(1,1-Dimethylpropyl)-phenyl]-2-methyl-propyl]-2,6-dimethylmorpholins und als wasserunlöslichen Filmbildner ein Copolymerisat aus Acryl- und Methacrylsäureestern mit einem geringen Gehalt an quartären Ammoniumgruppen verwendet. Derartige Copolymerisate sind z.B. aus der US Patentschrift 2677679 und der DAS 1617751, und antimykotisch wirksame Substanzen aus der DAS 2752 096, bekannt.

Zweckmässig ist der Gehalt an quartären Ammoniumgruppen im Copolymerisat derart, dass das molare Verhältnis der Ammoniumgruppen zu den übrigen neutralen (keine Ammoniumgruppen aufweisenden) Acrylsäure- bzw. Methacrylsäureestern (-COOR² in Formel I unten) zwischen etwa 1:20 und etwa 1:40 liegt.

Das mittlere Molekulargewicht eines derartigen Copolymerisats beträgt etwa 150'000. Derartige Copolymerisate können durch die folgende Teilformel charakterisiert werden, in welcher R₁ Wasserstoff oder Methyl und R₂ Methyl oder Aethyl bedeutet 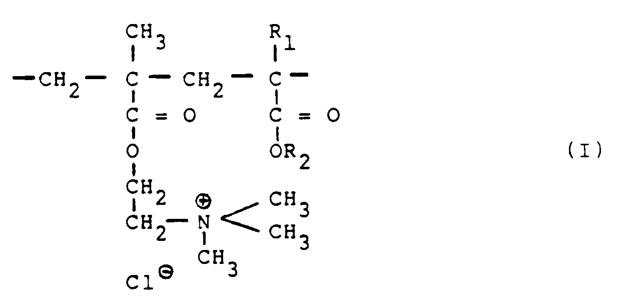
Derartige Substanzen sind als 12,5%ige Lacklösungen sowie als lösungsmittelfreie Festsubstanzen unter der Bezeichnung EUDRAGIT® RL und RS im Handel erhältlich. (s. Römpp's Chemielexikon, 8. Auflage, S. 1211).

Derartige Substanzen können je nach den besonderen Bedürfnissen auch miteinander vermischt werden.

Die Verwendung derartiger Copolymerisate gewährleistet aufgrund ihrer Quellbarkeit und Durchlässigkeit eine hohe Diffusionsrate und eine hohe Permeabilitätsrate für den Wirkstoff.

Die Verwendung derartiger Copolymerisate gewährleistet ferner neben den oben genannten Eigenschaften eine hohe Resistenz des Lacküberzuges gegen mechanische Verletzungen und gegen das Abwaschen, und zwar im gesamten pH-Bereich. Dies ermöglicht es, dass der die antimykotisch wirksame Substanz enthaltende Lackfilm während mehreren Tagen am Nagel verbleiben kann und daher der Zeitraum zwischen zwei Auftragungen der Lacklösung mehrere Tage beträgt.

Zweckmässig sind im erfindungsgemässen Präparat das Copolymerisat und die antimykotisch wirksame Substanz und allfällige weitere Zusatzstoffe, wie Weichmacher, Verdunstungsverzögerer und/oder Verdunstungsbeschleuniger in einem inerten organischen Lösungsmittel, insbesondere in Aethanol oder Methylenchlorid, gelöst.

Als Weichmacher können genannt werden: Glycerinacetate wie Triacetin, Phthalate, oder Weichmacher auf Kampferbasis. Neben den bevorzugten Lösungsmitteln, nämlich Aethanol und Methylenchlorid, können beispielsweise auch Aceton und Isopropanol verwendet werden.

Beispiele für Verdunstungsverzögerer sind Toluol, Butanol, Butylacetat, Amylalkohol und Amylacetat und als Beispiel für einen Verdunstungsbeschleuniger sei Aethylacetat genannt.

Die im erfindungsgemässen Präparat enthaltene antimykotisch wirksame Substanz ist das 4-[3-[p-(α,α-Dimethyl-benzyl)-phenyl]-2-methyl-propyl] -2,6-dimethyl-morpholin sein, oder das 4-[3-[p-(1,1-Dimethylpropyl)-phenyl]-2-methyl-propyl]-2,6-dimethylmorpholin, jeweils in Salzform, und zwar insbesondere in Form des Hydrochlorids oder des Nitrats. Bevorzugt werden die cis-Isomeren dieser Verbindungen verwendet. Diese cis-Isomeren sind aus der Europäischen Patentpublikation Nr. 24 334 bekannt, und zwar die zweitgenannte Verbindung unter der Bezeichnung 4-[3-(p-tert.Amyl-phenyl)-2-methylpropyl]-2,6-dimethyl-morpholin. Die Verwendung des cis-Isomeren dieser Verbindung, und zwar insbesondere in Form des Hydrochlorids, ist besonders bevorzugt.

Eine besonders bevorzugte Ausführungsform des erfindungsgemässen Präparats enthält die antimykotisch wirksame Substanz, also das oben genannte Morpholinderivat, in einer Menge von etwa 0,25 bis etwa 10 Gew.% insbesondere in einer Menge von etwa 5 Gew.%, bezogen auf die Base.

Gemäss einer zweckmässigen Ausführungsform des erfindungsgemässen Präparates enthält dieses das wasserunlösliche Copolymerisat in einer Menge von etwa 2-30 Gew.%, vorzugsweise 10-20 Gew.%, insbesondere etwa 12.5 Gew.%.

Das erfindungsgemässe Verfahren zur Herstellung des Präparates besteht darin, dass man das Copolymerisat in gelöster Form mit dem Wirkstoff und allfälligen Zusatzstoffen, wie Weichmacher, Verdunstungsbeschleuniger, Verdunstungsverzögerer und dergleichen mischt.

### Beispiel 1

Ein erfindungsgemässes Präparat, welches Methylenchlorid als Lösungsmittel enthält, hat die folgende Zusammensetzung:

| | |
|---|---|
| Wirkstoff | 5,574 g |
| EUDRAGIT® RL 100 | 12,5 g |
| Triacetin | 2,5 g |
| Butylacetat | 15,0 g |
| Methylenchlorid | ad 100,0 ml |

### Beispiel 2

Ein erfindungsgemässes Präparat, welches als Lösungsmittel Aethanol enthalt, weist die folgende Zusammensetzung auf:

| | |
|---|---|
| Wirkstoff | 5,574 g |
| EUDRAGIT® RL 100 | 12,5 g |
| Butylacetat | 5,0 g |
| Aethylacetat | 15,0 g |
| Aethanol | ad 100,0 ml |

Der in den obigen Beispielen verwendete Wirkstoff ist das cis-4-[3-[p-(1,1-Dimethylpropyl)-phenyl]-2-methyl-propyl]-2,6-dimethyl-morpholin-hydrochlorid oder das cis-4-[3-[p-(α,α-Dimethyl-benzyl)-phenyl]-2-methyl-propyl]-2,6-dimethyl-morpholin-hydrochlorid, und EUDRAGIT® RL 100 ist ein Copolymerisat aus Acryl- und Methacrylsäureestern, welches Ammoniumgruppen in einem molaren Verhältnis von 1:20 enthält (s. Firmenprospekt von Röhm Pharma GMBH, Weiterstadt, Bundesrepublik Deutschland: "EUDRAGIT® RL und RS Anwendung in der Arzneimittelherstellung" 1982). Anstelle dieses Copolymerisats können auch andere strukturell ähnliche Copolymerisate verwendet werden, z.B. ein Copolymerisat aus Trimethyl-methacryloxyäthylammoniumchlorid, Methacrylsäuremethylester und Acrylsäureäthylester mit einem geringen Gehalt an Ammoniumgruppen.

## Patentansprüche (Patentansprüche für folgende(n) Vertragsstaat(en): AT, BE, CH, DE, DK, FR, GB IT, LI, LU, NL, SE)

1. Nagellack, enthaltend mindestens eine antimykotisch wirksame Substanz und mindestens einen wasserunlöslichen Filmbildner, dadurch gekennzeichnet, dass die antimykotisch wirksame Substanz ein Salz des 4-[3-[p-(α,α-Dimethyl-benzyl)-phenyl]-2-methyl-propyl]-2,6-dimethyl-morpholins oder des 4-[3-[p-(1,1-Dimethylpropyl)-phenyl]-2-methyl-propyl]-2,6-dimethylmorpholins und der wasserunlösliche Filmbildner ein Copolymerisat aus Acryl- und Methacrylsäureestern mit einem geringen Gehalt an quartären Ammoniumgruppen ist.

2. Nagellack nach Anspruch 1, dadurch gekennzeichnet, dass das molare Verhältnis der Ammoniumgruppen zu den übrigen neutralen Acrylsäure- bzw. Methacrylsäureestern im Copolymerisat zwischen etwa 1:20 und etwa 1:40 liegt.

3. Nagellack nach Anspruch 1 oder 2, dadurch gekennzeichnet, dass das mittlere Molekulargewicht des Copolymerisats etwa 150'000 beträgt.

4. Nagellack nach Anspruch 1, 2 oder 3, dadurch gekennzeichnet, dass das Copolymerisat und die antimykotisch wirksame Substanz und allfällige weitere Zusatzstoffe, wie Weichmacher, Verdunstungsverzögerer und/oder Verdunstungsbeschleuniger, in einem inerten organischen Lösungsmittel, insbesondere in Aethanol oder Methylenchlorid, gelöst sind.

5. Nagellack nach einem der Ansprüche 1-4, dadurch gekennzeichnet, dass er als antimykotisch wirksame Substanz cis-4-[3-[p-(α,α-Dimethyl-benzyl)-phenyl]-2-methyl-propyl]-2,6-dimethyl-morpholin-hydrochlorid enthält.

6. Nagellack nach einem der Ansprüche 1-4, dadurch gekennzeichnet, dass er als antimykotisch wirksame Substanz cis-4-[3-[p-(1,1-Dimethylpropyl)-phenyl]-2-methyl-propyl]-2,6-dimethyl-morpholin-hydrochlorid enthält.

7. Nagellack nach einem der Ansprüche 1-6, dadurch gekennzeichnet, dass er die antimykotisch wirksame Substanz in einer Menge von etwa 0,25 bis etwa 10 Gew.%, insbesondere 5 Gew.%, bezogen auf die Base, enthält.

8. Nagellack nach einem der Ansprüche 1-7, dadurch gekennzeichnet, dass er das wasserunlösliche Copolymerisat in einer Menge von etwa 2-30 Gew.%, vorzugsweise 10-20 Gew.%, insbesondere etwa 12,5 Gew.% enthält.

9. Verfahren zur Herstellung eines Nagellacks nach einem der Ansprüche 1-8, dadurch gekennzeichnet, dass man das Copolymerisat in gelöster Form mit dem Wirkstoff und allfälligen Zusatzstoffen, wie Weichmacher, Verdunstungsbeschleuniger, Verdunstungsverzögerer und dergleichen vermischt.

10. Verfahren nach Anspruch 9, dadurch gekennzeichnet, dass man als Copolymerisat ein solches verwendet, in welchem das molare Verhältnis der Ammoniumgruppen zu den übrigen neutralen Acrylsäure- bzw. Methacrylsäureestern im Copolymerisat zwischen etwa 1:20 und etwa 1:40 liegt.

11. Verfahren nach Anspruch 9 oder 10, dadurch gekennzeichnet, dass das mittlere Molekulargewicht des verwendeten Copolymerisats etwa 150'000 beträgt.

12. Verfahren nach Anspruch 9, 10 oder 11, dadurch gekennzeichnet, dass man als antimykotisch wirksame Substanz das cis-4-[3-[p-(α,α-Dimethyl-benzyl)-phenyl]-2-methyl-propyl] -2,6-dimethyl-morpholin-hydrochlorid verwendet.

13. Verfahren nach Anspruch 9, 10 oder 11, dadurch gekennzeichnet, dass man als antimykotisch wirksame Substanz das cis-4-[3-[p-(1,1-Dimethylpropyl)-phenyl]-2-methyl-propyl]-2,6-dimethylmorpholin-hydrochlorid verwendet.

14. Verfahren nach einem der Ansprüche 9-13, dadurch gekennzeichnet, dass man die antimykotisch wirksame Substanz in einer Menge von etwa 0,25 bis etwa 10 Gew.%, insbesondere 5 Gew.%, bezogen auf die Base, verwendet.

15. Verfahren nach einem der Ansprüche 9-14, dadurch gekennzeichnet, dass man das Copolymerisat in einer Menge von etwa 2-30 Gew.%, vorzugsweise 10-20 Gew.%, insbesondere etwa 12,5 Gew.%, verwendet.

## Patentansprüche (Patentansprüche für folgende(n) Vertragsstaat(en): GR, ES)

1. Verfahren zur Herstellung eines Nagellacks, welcher mindestens eine antimykotisch wirksame Substanz und mindestens einen wasserunlöslichen Filmbildner enthält, dadurch gekennzeichnet, dass man als antimykotisch wirksame Substanz ein Salz des 4-[3-[p-(α,α-Dimethyl-benzyl)-phenyl]-2-methyl-propyl]-2,6-dimethyl-morpholins oder des 4-[3-[p-(1,1-Dimethylpropyl)-phenyl]-2-methyl-propyl]-2,6-dimethylmorpholins, und als wasserunlöslichen Filmbildner ein Copolymerisat aus Acryl- und Methacrylsäureestern mit einem geringen Gehalt an quartären Ammoniumgruppen verwendet, und dass man das Copolymerisat in gelöster Form mit der antimykotisch wirksamen Substanz und allfälligen Zusatzstoffen, wie Weichmacher, Verdunstungsbeschleuniger, Verdunstungsverzögerer und dergleichen, vermischt.

2. Verfahren nach Anspruch 1, dadurch gekennzeichnet, dass man als Copolymerisat ein solches verwendet, in welchem das molare Verhältnis der Ammoniumgruppen zu den übrigen neutralen Acrylsäure- bzw. Methacrylsäureestern im Copolymerisat zwischen etwa 1:20 und etwa 1:40 liegt.

3. Verfahren nach Anspruch 1 oder 2, dadurch gekennzeichnet, dass das mittlere Molekulargewicht des verwendeten Copolymerisats etwa 150'000 beträgt.

4. Verfahren nach Anspruch 1, 2 oder 3, dadurch gekennzeichnet, dass man als antimykotisch wirksame Substanz das cis-4-[3-[p-(α,α-Dimethyl-benzyl)-phenyl]-2-methyl-propyl]-2,6-dimethyl-morpholin-hydrochlorid verwendet.

5. Verfahren nach Anspruch 1, 2 oder 3, dadurch gekennzeichnet, dass man als antimykotisch wirksame Substanz das cis-4-[3-[p-(1,1-Dimethylpropyl)-phenyl]-2-methylpropyl]-2,6-dimethylmorpholin-hydrochlorid verwendet.

6. Verfahren nach einem der Ansprüche 1-5, dadurch gekennzeichnet, dass man die antimykotisch wirksame Substanz in einer Menge von etwa 0,25 bis etwa 10 Gew.%, insbesondere 5 Gew.%, bezogen auf die Base, verwendete.

7. Verfahren nach einem der Ansprüche 1-6, dadurch gekennzeichnet, dass man das Copolymerisat in einer Menge von etwa 2-30 Gew.%, vorzugsweise 10-20 Gew.%, insbesondere etwa 12,5 Gew.%, verwendet.

## Claims (Claims for the following Contracting State(s): AT, BE, CH, DE, DK, FR, GB, IT, LI LU, NL, SE)

1. A nail varnish containing at least one antimycotically-active substance and at least one water-insoluble film-former, characterized in that the antimycotically-active substance is a salt of 4-[3-[p-(α,α-dimethyl-benzyl)-phenyl]-2-methyl-propyl]-2,6-dimethyl-morpholine or of 4-[3-[p-(1,1-dimethylpropyl)-phenyl]-2-methyl-propyl]-2,6-dimethylmorpholine and the water-insoluble film-former is a copolymerizate of acrylic acid esters and methacrylic acid esters having a low content of quaternary ammonium groups.

2. A nail varnish according to claim 1, characterized in that the molar ratio of ammonium groups to the remaining neutral acrylic acid esters and, respectively, methacrylic acid esters in the copolymerizate lies between about 1:20 and about 1:40.

3. A nail varnish according to claim 1 or 2, characterized in that the average molecular weight of the copolymerizate amounts to about 150,000.

4. A nail varnish according to claim 1, 2 or 3, characterized in that the copolymerizate and the antimycotically-active substance and any further additives such as plasticizers, evaporation retarders and/or evaporation accelerators are dissolved in an inert organic solvent, especially in ethanol or methylene chloride.

5. A nail varnish according to any one of claims 1-4, characterized in that it contains cis-4-[3-[p-(α,α-dimethyl-benzyl)-phenyl]-2-methyl-propyl]-2,6-dimethyl-morpholine hydrochloride as the antimycotically-active substance.

6. A nail varnish according to any one of claims 1-4, characterized in that it contains cis-4-[3-[p-(1,1-dimethylpropyl)-phenyl]-2-methyl-propyl]-2,6-dimethyl-morpholine hydrochloride as the antimycotically-active substance.

7. A nail varnish according to any one of claims 1-6, characterized in that it contains the antimycotically-active substance in an amount of about 0.25 to about 10 wt.%, especially 5 wt.%, based on the base.

8. A nail varnish according to any one of claims 1-7, characterized in that it contains the water-insoluble copolymerizate in an amount of about 2-30 wt.%, preferably 10-20 wt.%, especially about 12.5 wt.%.

9. A process for the manufacture of a nail varnish according to any one of claims 1-8, characterized by mixing the copolymerizate in dissolved form with the active substance and any additives such as plasticizers, evaporation accelerators, evaporation retarders and the like.

10. A process according to claim 9, characterized in that there is used a copolymerizate in which the molar ratio of ammonium groups to the remaining neutral acrylic acid esters and, respectively, methacrylic acid esters in the copolymerizate lies between about 1:20 and about 1:40.

11. A process according to claim 9 or 10, characterized in that the average molecular weight of the copolymerizate used amounts to about 150,000.

12. A process according to claim 9, 10 or 11, characterized in that cis-4-[3-[p-(α,α-dimethyl-benzyl)-phenyl]-2-methyl-propyl]-2,6-dimethyl-morpholine hydrochloride is used as the antimycotically-active substance.

13. A process according to claim 9, 10 or 11, characterized in that cis-4-[3-[p-(1,1-dimethylpropyl)-phenyl]-2-methylpropyl]-2,6-dimethylmorpholine hydrochloride is used as the antimycotically-active substance.

14. A process according to any one of claims 9-13, characterized in that the antimycotically-active substance is used in an amount of about 0.25 to about 10 wt.%, especially 5 wt.%, based on the base.

15. A process according to any one of claims 9-14, characterized in that the copolymerizate is used in an amount of about 2-30 wt.%, preferably 10-20 wt.%, especially about 12.5 wt.%.

## Claims (Claims for the following Contracting State(s): GR, ES)

1. A process for the manufacture of a nail varnish which contains at least one antimycotically-active substance and at least one water-insoluble film-former, characterized in that a salt of 4-[3-[p-(α,α-dimethyl-benzyl)-phenyl]-2-methyl-propyl]-2,6-dimethyl-morpholine or of 4-[3-[p-(1,1-dimethylpropyl)-phenyl]-2-methyl-propyl]-2,6-dimethylmorpholine is used as the antimycotically-active substance and a copolymerizate of acrylic acid esters and methacrylic acid esters having a low content of quaternary ammonium groups is used as the water-insoluble film-former and in that the copolymerizate in dissolved form is mixed with the antimycotically-active substance and optionally with additives such as plasticizers, evaporation accelerators, evaporation retarders and the like.

2. A process according to claim 1, characterized in that there is used a copolymerizate in which molar ratio of ammonium groups to the remaining neutral acrylic acid esters and, respectively, methacrylic acid esters in the copolymerizate lies between about 1:20 and about 1:40.

3. A process according to claim 1 or 2, characterized in that the average molecular weight of the copolymerizate used amounts to about 150,000.

4. A process according to claim 1, 2 or 3, characterized in that cis-4-[3-[p-(α,α-dimethyl-benzyl)-phenyl]-2-methylpropyl]-2,6-dimethyl-morpholine hydrochloride is used as the antimycotically-active substance.

5. A process according to claim 1, 2 or 3, characterized in that cis-4-[3-[p-(1,1-dimethylpropyl)-phenyl]-2-methylpropyl]-2,6-dimethylmorpholine hydrochloride is used as the antimycotically-active substance.

6. A process according to any one of claims 1-5, characterized in that the antimycotically-active substance is used in an amount of about 0.25 to about 10 wt.%, especially 5 wt.%, based on the base.

7. A process according to any one of claims 1-6, characterized in that the copolymerizate is used in an amount of about 2-30 wt.%, preferably 10-20 wt.%, especially about 12.5 wt.%.

## Revendications (Revendications pour l'(les) Etat(s) contractant(s) suivant(s): AT, BE, CH, DE, DK, FR, GB, IT, LI, LU, NL, SE)

1. Vernis à ongles contenant au moins une substance à action antimycotique et au moins une matière filmogène insoluble dans l'eau, caractérisé en ce que la substance à action antimycotique est un sel de la 4-[3-[p-(α,α-diméthylbenzyl)-phényl]-2-méthylpropyl]-2,6-diméthylmorpholine ou de la 4-[3-[p-(1,1-diméthylpropyl)-phényl]-2-méthylpropyl]-2,6-diméthylmorpholine et la matière filmogène insoluble dans l'eau est un copolymère d'esters d'acides acrylique et méthacrylique avec une faible teneur en groupes ammonium quaternaires.

2. Vernis à ongles selon la revendication 1, caractérisé en ce que le rapport molaire des groupes ammonium aux autres esters neutres d'acides acrylique ou méthacrylique dans le copolymère se situe entre environ 1:20 et environ 1:40.

3. Vernis à ongles selon la revendication 1 ou 2, caractérisé en ce que le poids moléculaire moyen du copolymère s'élève à environ 150.000.

4. Vernis à ongles selon la revendication 1, 2 ou 3, caractérisé en ce que le copolymère et la substance à action antimycotique ainsi que les autres additifs éventuels, comme les plastifiants, les retardateurs d'évaporation et/ou accélérateurs d'évaporation, sont dissous dans un solvant organique inerte, en particulier dans l'éthanol ou le chlorure de méthylène.

5. Vernis à ongles selon l'une des revendications 1 à 4, caractérisé en ce qu'il contient comme substance à action antimycotique le chlorhydrate de cis-4-[3-[p-(α,α-diméthyl-benzyl)-phényl]-2-méthylpropyl]-2,6-diméthylmorpholine.

6. Vernis à ongles selon l'une des revendications 1 à 4, caractérisé en ce qu'il contient comme substance à action antimycotique le chlorhydrate de cis-4-[3-[p-(1,1-diméthylpropyl)-phényl]-2-méthylpropyl]-2,6-diméthyl morpholine.

7. Vernis à ongles selon l'une des revendications 1 à 6, caractérisé en ce qu'il contient la substance à action antimycotique en une quantité d'environ 0,25 à environ 10 % en poids, en particulier 5 % en poids, par rapport à la base.

8. Vernis à ongles selon l'une des revendications 1 à 7, caractérisé en ce qu'il contient le copolymère insoluble dans l'eau en une quantité d'environ 2-30 % en poids, de préférence 10-20 % en poids, en particulier d'environ 12,5 % en poids.

9. Procédé de préparation d'un vernis à ongles selon l'une des revendications 1 à 8, caractérisé en ce qu'on mélange le copolymère sous forme dissoute avec la matière active et les additifs éventuels, comme les plastifiants, les accélérateurs d' évaporation, les retardateurs d'évaporation, etc.

10. Procédé selon la revendication 9, caractérisé en ce qu'on utilise comme copolymère un corps dans lequel le rapport molaire des groupes ammonium aux autres esters neutres d'acides acrylique ou méthacrylique dans le copolymère se situe entre environ 1:20 et environ 1:40.

11. Procédé selon la revendication 9 ou 10, caractérisé en ce que le poids moléculaire moyen du copolymère utilisé s'élève à environ 150.000.

12. Procédé selon la revendication 9, 10 ou 11, caractérisé en ce qu'on utilise comme substance à action antimycotique le chlorhydrate de cis-4-[3-[p-(α,α-diméthylbenzyl)-phényl]-2-méthylpropyl]-2,6-diméthylmorpholine.

13. Procédé selon la revendication 9, 10 ou 11, caractérisé en ce qu'on utilise comme substance à action antimycotique le chlorhydrate de cis-4-[3-[p-(1,1-diméthylpropyl)-phényl]-2-méthylpropyl]-2,6-diméthylmorpholine.

14. Procédé selon l'une quelconque des revendications 9 à 13, caractérisé en ce qu'on utilise la substance à action antimycotique en une quantité d'environ 0,25 à environ 10 % en poids, en particulier 5 % en poids, par rapport à la base.

15. Procédé selon l'une quelconque des revendications 9 à 14, caractérisé en ce qu'on utilise le copolymère en une quantité d'environ 2-30 % en poids, de préférence 10-20 % en poids, en particulier d'environ 12,5 % en poids.

## Revendications (Revendications pour l'(les) Etat(s) contractant(s) suivant(s): GR, ES)

1. Procédé de préparation d'un vernis à ongles contenant au moins une substance à action antimycotique et au moins une matière filmogène insoluble dans l'eau, caractérisé en ce qu'on utilise comme substance à action antimycotique un sel de la 4-[3-[p-(α,α-diméthyl-benzyl)-phényl]-2-méthylpropyl]-2,6-diméthylmorpholine ou de la 4-[3-[p-(1,1-diméthylpropyl)-phényl]-2-méthylpropyl]-2,6-diméthylmorpholine, et comme matière filmogène insoluble dans l'eau un copolymère d'esters d'acides acrylique et méthacrylique avec une faible teneur en groupes ammonium quaternaires, et en ce qu'on mélange le polymère sous forme dissoute avec la substance à action antimycotique et les additifs éventuels, comme des plastifiants, des accélérateurs d' évaporation, des retardateurs d'évaporation, etc.

2. Procédé selon la revendication 1, caractérisé en ce qu'on utilise comme copolymère un corps dans lequel le rapport molaire des groupes ammonium aux autres esters neutres d'acides acrylique ou méthacrylique dans le copolymère se situe entre environ 1:20 et environ 1:40.

3. Procédé selon la revendication 1 ou 2, caractérisé en ce que le poids moléculaire moyen du copolymère utilisé s'élève à environ 150.000.

4. Procédé selon la revendication 1, 2 ou 3, caractérisé en ce qu'on utilise comme substance à action antimycotique le chlorhydrate de cis-4-[3-[p-(α,α-diméthylbenzyl)-phényl]-2-méthylpropyl]-2,6-diméthylmorpholine.

5. Procédé selon la revendication 1, 2 ou 3, caractérisé en ce qu'on utilise comme substance à action antimycotique le chlorhydrate de cis-4-[3-[p-(1,1-diméthylpropyl)-phényl]-2-méthylpropyl]-2,6-diméthylmorpholine.

6. Procédé selon l'une des revendications 1 à 5, caractérisé en ce qu'on utilise la substance à action antimycotique en une quantité d'environ 0,25 à environ 10 % en poids, en particulier 5 % en poids, par rapport à la base.

7. Procédé selon l'une des revendications 1 à 6, caractérisé en ce qu'on utilise le copolymère en une quantité d'environ 2-30 % en poids, de préférence de 10-20 % en poids, en particulier d'environ 12,5 % en poids.
